# EUROPEAN PATENT APPLICATION

(11) **EP 3 885 038 A1**
(43) Date of publication of application: **29.09.2021**
(21) Application number: 20166176.6
(22) Date of filing: 27.03.2020
(51) Int. Cl.: B01J 23/50, C07D 301/00, B01J 37/02

(54) **PROCESS FOR PRODUCING AN EPOXIDATION CATALYST**

(71) Applicant: BASF SE, 67056 Ludwigshafen am Rhein (DE)
(72) Inventor: KARPOV, Andrey, 67056 Ludwigshafen (DE)
(74) Representative: Reitstötter Kinzebach

(57) **Abstract**

A process for producing an epoxidation catalyst, comprising: i) placing a porous support under reduced pressure, the porous support having an Hg pore volume in the range of 0.4 to 3.0 mL/g, as determined by mercury porosimetry; ii) gradually adding to the porous support a volume of aqueous silver impregnation solution over a period of at least 15 min at a temperature of 40 to 150 °C, while maintaining reduced pressure; the volume of aqueous silver impregnation solution added per gram of porous support being greater than the Hg pore volume of the porous support; iii) maintaining the reduced pressure to remove volatiles to a weight loss of at least 3 wt.-%, relative to the weight of aqueous silver impregnation solution; iv) calcining the impregnated porous support at a temperature of 200 to 800 °C. The process allows for a uniform distribution of silver with a high degree of silver dispersion. The invention further relates to a process for producing an alkylene oxide by gas-phase oxidation of an alkylene, comprising reacting an alkylene and oxygen in the presence of the epoxidation catalyst.

## Description

The present invention relates to a process for producing an epoxidation catalyst, that is a catalyst effective in the oxidative conversion of an alkylene to an alkylene oxide, and a process for producing an alkylene oxide by gas-phase oxidation of an alkylene by means of oxygen in the presence of the catalyst.

Alkylene oxides, in particular ethylene oxide, are produced in large volume and are primarily used as intermediates in the production of several industrial chemicals. In the industrial oxidation of ethylene to ethylene oxide, e.g., heterogeneous catalysts comprising silver are used. To carry out the heterogeneously catalyzed gas-phase oxidation, a mixture of ethylene and an oxygen-comprising gas, such as air or pure oxygen, is generally passed through a plurality of tubes with a packing of shaped catalyst bodies, wherein the plurality of tubes is located in a reactor.

Catalysts containing high levels of silver, such as, e.g., at least 20 wt.-% of silver, are often able to increase the activity, stability and/or work rate of the catalyst, as compared to a similar catalyst not having the same high silver concentration.

Typically, catalysts comprising silver are prepared by impregnating a porous support with an impregnation solution, subsequently drying the impregnated support and calcining the dried, impregnated support at high temperatures so as to obtain the silver-containing catalyst.

DE 39 37 247 A1 describes a process for producing a silver-containing catalyst for producing ethylene oxide, wherein a support is contacted with a silver impregnation solution, and reduced pressure and heat are subsequently applied to the mixture so as to remove solvent from the impregnation solution. During solvent removal, the pressure is increased to atmospheric pressure in regular intervals. The impregnated, pre-dried support is dried at elevated temperatures and subsequently calcined to obtain a silver-containing catalyst.

It is an object of the present invention to provide an efficient process for producing catalysts comprising high amounts of silver.

The invention relates to a process for producing an epoxidation catalyst, comprising:
i) placing a porous support under reduced pressure, the porous support having an Hg pore volume in the range of 0.4 to 3.0 mL/g, as determined by mercury porosimetry;
ii) gradually adding to the porous support a volume of aqueous silver impregnation solution over a period of at least 15 min at a temperature of 40 to 150 °C, while maintaining reduced pressure; the volume of aqueous silver impregnation solution added per gram of porous support being greater than the Hg pore volume of the porous support;
iii) maintaining the reduced pressure to remove volatiles to a weight loss of at least 3 wt.-%, relative to the weight of aqueous silver impregnation solution;
iv) calcining the impregnated porous support at a temperature of 200 to 800 °C, so as to convert a silver compound deposited on the porous support to elemental silver.

The silver impregnation solution penetrates into the pores of the support by absorption and/or capillary action. Whereas the total quantity of the silver impregnation solution used to impregnate the porous support is more than is necessary to fill the pore volume of the porous support, by gradual addition of the solution and concurrent removal of volatiles under reduced pressure and elevated temperature, all of the solution is absorbed by the support. During impregnation, there is preferably no excess of aqueous silver impregnation solution at any time, i.e. no non-absorbed aqueous silver impregnation solution at any time. It is believed that an excess of silver impregnation solution leads to a layer of increased silver concentration on the support's surface. This may be attributed to a change of the solution transport from a capillary action process to a diffusion process. The present process allows for a uniform distribution of silver with a high degree of silver dispersion.

The porous support usually comprises a high proportion of alumina, i.e. Al₂O₃, and in particular alpha-alumina, for example at least 50 wt.-%, at least 70 wt.-%, or at least 90 wt.-%, preferably at least 95 wt.-%, most preferably at least 97.5 wt.-%, based on the total weight of the support. Besides alumina, the porous alumina support may comprise other components, for example binders such as silicates, or other refractory oxides such as zirconia or titania.

The porous support preferably comprises individual shaped bodies. The size and shape of the individual shaped bodies and thus of the catalyst is selected to allow a suitable packing of the shaped bodies in a reactor tube. The shaped bodies suitable for the catalysts of the invention are preferably used in reactor tubes with a length from 6 to 14 m and an inner diameter from 20 mm to 50 mm. In general, the support is comprised of individual bodies having a maximum extension in the range of 3 to 20 mm, such as 4 to 15 mm, in particular 5 to 12 mm. The maximum extension is understood to mean the longest straight line between two points on the outer circumference of the support.

The shape of the support is not especially limited, and may be in any technically feasible form, depending, e.g., on the extrusion process. For example, the support may be a solid extrudate or a hollow extrudate, such as a hollow cylinder. In another embodiment, the support may be characterized by a multilobe structure. A multilobe structure is meant to denote a cylinder structure which has a plurality of void spaces, e.g., grooves or furrows, running in the cylinder periphery along the cylinder height. Generally, the void spaces are arranged essentially equidistantly around the circumference of the cylinder. Preferably, the support is in the shape of a solid extrudate, such as pellets or cylinders, or a hollow extrudate, such as a hollow cylinder. Alternatively, the support may be shaped by tableting.

The porous support has a total Hg pore volume in the range of 0.4 to 3.0 mL/g, preferably 0.45 to 1.0 mL/g, more preferably 0.5 to 0.7 mL/g, as determined by mercury porosimetry. Mercury porosimetry may be performed using a Micrometrics AutoPore IV 9500 mercury porosimeter (140 degrees contact angle, 485 dynes/cm Hg surface tension, 60000 psia max head pressure). The Hg porosity is determined according to DIN 66133 herein, unless stated otherwise. It is believed that a Hg pore volume in this range allows for a favorable duration of exposure of the obtained ethylene oxide to the catalyst.

The water absorption of the porous support is typically in the range of 0.35 to 0.70 mL/g (mL of water/gram of support). Preferably, the water absorption of the porous support is in the range of 0.38 to 0.65 mL/g, most preferably 0.41 to 0.60 mL/g. Water absorption refers to vacuum cold water uptake measured at a vacuum of 80 mbar. Vacuum cold water uptake is determined by placing about 100 g of support ("initial support weight") in a rotating flask, covering the support with deionized water, and rotating the rotary evaporator for 5 min at about 30 rpm. Subsequently, a vacuum of 80 mbar is applied for 3 min, the water and the support are transferred into a glass funnel, and the support is kept in the funnel for about 5 min with occasional shaking in order to ensure that adhering water runs down the funnel. The support is weighed ("final support weight"). The water absorption is calculated by subtracting the initial support weight from the final support weight and then dividing this difference by the initial support weight.

The porous support generally has a BET surface area of 0.5 to 10 m²/g, preferably 1.0 to 5.0 m²/g, more preferably 1.5 to 3.0 m²/g. The BET method is a standard, well-known method and widely used method in surface science for the measurements of surface areas of solids by physical adsorption of gas molecules. The BET surface is determined according to DIN ISO 9277 herein, unless stated otherwise.

The porous support may comprise impurities, such as sodium, potassium, iron, silica, magnesium, calcium, zirconium in an amount of 100 to 10000 ppm, based on the total weight of the support.

The porous support preferably does not have wash-coat particles or a wash-coat layer on its surface, so as to fully maintain the porosity and BET surface area of the uncoated support.

According to step i) of the inventive process, a porous support is placed under reduced pressure prior to adding a silver impregnation solution to the porous support. All pressures described herein are understood to be absolute pressures, unless noted otherwise.

Preferably, the porous support is placed under a pressure of at most 800 mbar, at most 600 mbar, at most 400 mbar, at most 200 mbar or at most 150 mbar in step i) of the inventive process, and said pressure is maintained in steps ii) and iii). Typically, the applied pressure is at least 5 mbar, at least 10 mbar, at least 20 mbar, at least 35 mbar or at least 50 mbar. Thus, the porous support is typically placed under a pressure of 5 to 800 mbar, 10 to 600 mbar, 20 to 400 mbar, 35 to 200 mbar or 50 to 150 mbar.

According to step ii) of the inventive process, the aqueous silver impregnation solution is added to the porous support over a period of at least 15 min, such as at least 30 min or at least 45 min at a temperature of 40 to 150 °C, such as and most preferably 45 to 100 °C, such as 45 to 75 °C or 50 to 60 °C. The reduced pressure is maintained during addition of the silver impregnation solution to the porous support, which is believed to further contribute to a high degree of penetration of the pores of the support with the silver impregnation solution, thus allowing for a high degree of dispersion of the silver deposited in the pores of the support.

During step ii), the temperature of 40 to 150 °C may be maintained by continuously heating the porous support. For this purpose, an impregnation vessel with a heating jacket, such as a steam jacket, may be used. Any other heat-exchange medium such as warm water can also be used.

In one embodiment, the impregnation solution is be pre-heated. Notably, due to safety considerations, the impregnation solution is preferably not heated to a temperature of more than 120 °C, more preferably not more than 100 °C, and in particular not more than 80 °C. Suitably, hold-up of the pre-heated impregnation solution is kept to a minimum. Thus, the impregnation solution is preferably pre-heated in a flow-heater immediately before being added to the porous support.

Any silver impregnation solution suitable for impregnating a refractory support known in the art can be used. Silver impregnation solutions typically contain silver oxalate, or a combination of silver oxide and oxalic acid, together in ethylenediamine. Suitable impregnation solutions are described in EP 0 716 884 A2, EP 1 115 486 A1, EP 1 613 428 A1, US 4,731,350, WO 2004/094055 A2, WO 2009/029419 A1, WO 2015/095508 A1, US 4,356,312, US 5,187,140, US 4,908,343, US 5,504,053 and WO 2014/105770 A1.

In the inventive process, the volume of aqueous silver impregnation solution added to the porous support per gram of porous support in step ii) is greater than the Hg pore volume of the porous support. It is understood that "the volume of aqueous silver impregnation solution" relates to the total amount of aqueous silver impregnation solution added in step ii) over the entire time period during which the aqueous silver impregnation solution is added. This implies that the added volume of aqueous silver impregnation solution is greater than the freely absorbable volume of the aqueous silver impregnation solution.

Preferably, the volume of aqueous silver impregnation solution added to the porous support per gram of porous support in step ii) is at least 103%, at least 105%, at least 110% or at least 115% of the Hg pore volume of the porous support, and at most 130%, at most 125% or at most 120% of the Hg pore volume of the porous support. Typically, the volume of aqueous silver impregnation solution added to the porous support per gram of porous support in step ii) is 103% to 130% of the Hg pore volume of the porous support, preferably 105% to 125%, 110% to 120% or 115% to 120%.

According to step iii) of the inventive process, the reduced pressure is maintained to remove volatiles to a weight loss of at least 3 wt.-%, relative to the weight of the aqueous silver impregnation solution. The "weight loss" is meant to denote the total weight of the support and silver impregnation solution minus the weight of the impregnated support. It is understood that the term "volatiles" refers to all components which are removed under the reduced pressure. For example, the aqueous silver impregnation solution comprises water, and may comprise one or more organic compounds. Water and organic compounds are susceptible to evaporation and/or boiling under reduced pressure and heat, and the resultant vapor is at least partially removed from the process under such conditions, leaving behind non-boiling components such as silver compounds that may precipitate out of the solution within the pores of the support.

The reduced pressure is maintained at least over the entire time period during which the aqueous silver impregnation solution is added, and optionally beyond this time period.

Preferably, the reduced pressure is maintained to remove volatiles to a weight loss of at least 5 wt.-%, at least 7 wt.-%, at least 10 wt.-%, at least 15 wt.-% or at least 20 wt.-%, relative to the weight of the aqueous silver impregnation solution. The weight loss is preferably large enough so that the volume of the silver impregnation solution added to the porous support per gram of porous support is reduced to being less than the Hg pore volume of the porous support. Advantageously, this allows for a concentration of the silver component of the silver impregnation solution on the surface of the porous support and within its pores. The thus impregnated support and the catalyst derived therefrom accordingly comprise an increased amount of silver.

On the other hand, the reduced pressure is typically maintained to remove volatiles to a weight loss of at most 35 wt.-%, at most 30 wt.-%, at most 25 wt.-% or at least 22 wt.-%, relative to the weight of the aqueous silver impregnation solution. It is believed that this advantageously allows for a homogenous distribution of silver on the porous support and within its pores.

In a preferred embodiment, the inventive process comprises pre-heating the porous support to a temperature of 40 to 200 °C, preferably 40 to 150 °C and most preferably 45 to 100 °C, such as 45 to 75 °C or 50 to 60 °C, prior to addition of the silver impregnation solution. Pre-heating the porous support allows for a faster removal of volatiles and thus a more efficient process. Pre-heating of the porous support can be performed after placing the porous support under reduced pressure or prior to placing the porous support under reduced pressure.

Preferably, the inventive process comprises agitation of the porous support during steps ii) and iii). The term "agitation" is understood to refer to a mechanical movement, e.g., shaking, vibrating or tumbling the porous support. Agitating the porous support allows for a more homogenous distribution of impregnation liquid on the support and a faster removal of volatiles.

Any impregnation apparatus suitable for impregnation under reduced pressure may be used, including double-cone blenders and free-fall mixing reactors such as tumble dryers.

According to step iv) of the inventive process, the impregnated porous support is calcined at a temperature of 200 to 800 °C, so as to convert a silver compound deposited on the porous support to elemental silver. Typically, the impregnated porous support is calcinated at atmospheric pressure (approximately 1013 mbar absolute) at a temperature of 200 to 800 °C, under inert gas flow, such as nitrogen flow, e.g., in a calcination oven.

Preferably, the calcination temperature is 220 to 650 °C, such as 240 to 500 °C or 260 to 350 °C. The impregnated porous support is typically subjected to calcination for a period of 5 to 60 min, such as 7 to 20 min. Further examples of calcination processes are described in the art, e.g., US 5,504,052, US 5,646,087, US 7,553,795, US 8,378,129, US 8,546,297, US2014/0187417, EP 1893331 or WO2012/140614.

In one embodiment, the impregnated porous support is allowed to cool to room temperature (about 23 °C) while maintaining reduced pressure prior to calcination. Subsequently, the impregnated porous support is placed under atmospheric pressure (approximately 1013 mbar absolute) and then calcined as described above.

Preferably, the impregnated porous support obtained in step iii) is calcined without further drying steps prior to calcination. In particular, it is preferred that the impregnated porous support is heated to a temperature of at least 200 °C over a period of at most 15 min, such as at most 10 min.

The catalyst obtained by the inventive process preferably comprises at least 15 wt.-% of silver, relative to the total weight of the catalyst. Preferably the catalyst has a content of at least 18 wt.-%, more preferably at least 20 wt.-%, such as 22 wt.-% or 25 wt.-%, relative to the total weight of catalyst.

For example, the catalyst may comprise 15 to 70 wt.-% of silver, relative to the total weight of the catalyst. A preferred catalyst comprises 20 to 60 wt.-% of silver, more preferably 20 to 50 wt.-% of silver, such as 25 to 35 wt.-%, relative to the total weight of the catalyst. A silver content in this range allows for a favorable balance between turnover induced by each catalyst body and cost-efficiency of producing the catalyst.

In order to obtain a catalyst having high silver contents, steps i) to iv) can be repeated. In this case, it is understood that the intermediate product obtained after the first (or subsequent up to the last but one) impregnation/calcination cycle comprises a part of the total amount of target silver and/or promoter concentrations. The intermediate product is then again impregnated with the silver impregnation solution and calcined to yield the target silver and/or promoter concentrations. Suitably, the volume(s) and concentration of aqueous silver impregnation solution may be adapted to yield the desired silver concentration.

In another embodiment, steps i) to iv) are repeated, with the exception that step ii) is modified in that the aqueous silver impregnation solution is added to the porous support at a temperature below 40 °C, preferably at a temperature of 20 to 25 °C.

If steps i) to iv) are repeated, it is preferable that the first implementation of calcination step iv) is performed while directing an oxygen-containing gas over the impregnated porous support, in particular while directing air over the impregnated porous support.

In another embodiment where steps i) to iv) are repeated, it is preferable that the final iteration of step iv) is performed while directing an inert gas flow, e.g. a nitrogen flow, over the impregnated porous support, while all previous iterations of step iv) are performed while directing an oxygen-containing gas over the impregnated porous support, in particular while directing air over the impregnated porous support.

Besides silver, the catalyst may comprise one or more promoting species ("promoter"). A promoting species denotes a component that provides an improvement in one or more of the catalytic properties of the catalyst when compared to a catalyst not containing said component. The promoting species can be any of those species known in the art that function to improve the catalytic properties of the silver catalyst. Examples of catalytic properties include operability (resistance to runaway), selectivity, activity, turnover and catalyst longevity.

The catalyst may comprise a promoting amount of a transition metal or a mixture of two or more transition metals. Suitable transition metals can include, for example, the elements from Groups IIIB (scandium group), IVB (titanium group), VB (vanadium group), VIB (chromium group), VIIB (manganese group), VIIIB (iron, cobalt, nickel groups), IB (copper group), and IIB (zinc group) of the Periodic Table of the Elements, as well as combinations thereof. More typically, the transition metal is an early transition metal, i.e., from Groups IIIB, IVB, VB or VIB, such as, for example, hafnium, yttrium, molybdenum, tungsten, rhenium, chromium, titanium, zirconium, vanadium, tantalum, niobium, or a combination thereof. In one embodiment, the transition metal promoter(s) is (are) present in a total amount from 150 ppm to 10000 ppm, typically 225 ppm to 7000 ppm, most typically from 300 ppm to 4000 ppm, expressed in terms of metal(s) relative to the total weight of the catalyst.

Of the transition metal promoters listed, rhenium (Re) is a particularly efficacious promoter for ethylene epoxidation high selectivity catalysts. The rhenium component in the catalyst can be in any suitable form, but is more typically one or more rhenium-containing compounds (e.g., a rhenium oxide) or complexes.

Preferably, the catalyst comprises 100 to 3000 ppm by weight of rhenium, relative to the total weight of the catalyst. It is preferred that the catalyst comprises 250 to 2000 ppm by weight of rhenium, more preferably 500 to 1500 ppm by weight of rhenium, relative to the total weight of the catalyst.

In some embodiments, the catalyst may include a promoting amount of an alkali metal or a mixture of two or more alkali metals. Suitable alkali metal promoters include, for example, lithium, sodium, potassium, rubidium, cesium or combinations thereof. The amount of alkali metal, e.g. potassium, will typically range from 50 ppm to 5000 ppm, more typically from 300 ppm to 2500 ppm, most typically from 500 ppm to 1500 ppm expressed in terms of the alkali metal relative to the total weight of the catalyst. The amount of alkali metal is determined by the amount of alkali metal contributed by the porous support and the amount of alkali metal contributed by the impregnation solution described below.

Combinations of heavy alkali metals like cesium (Cs) or rubidium (Rb) with light alkali metals like lithium (Li), sodium (Na) and potassium (K) are particularly preferred.

Cesium is an especially preferred alkali metal promoter. Preferably, the catalyst comprises 100 to 2000 ppm by weight of cesium, relative to the total weight of the catalyst. It is preferred that the catalyst comprises 400 to 1750 ppm by weight of cesium, more preferably 600 to 1500 ppm by weight of cesium, relative to the total weight of the catalyst.

Preferably the catalyst contains at least two light alkali metals, selected from sodium, potassium and lithium. Most preferably the catalyst contains sodium, potassium and lithium.

Preferably, the catalyst comprises 40 to 1170 ppm by weight of potassium, relative to the total weight of the catalyst. It is preferred that the catalyst comprises 100 to 1000 ppm by weight of potassium, most preferably 140 to 500 ppm by weight of potassium. The amount of potassium is determined by the amount of potassium contributed by the porous support and the amount of potassium contributed by the impregnation solution described below.

Preferably, the catalyst comprises 100 to 2000 ppm by weight of lithium, relative to the total weight of the catalyst. It is preferred that the catalyst comprises 150 to 1500 ppm by weight of lithium, most preferably 300 to 1000 ppm by weight of lithium. The amount of lithium is determined by the amount of lithium contributed by the porous support and the amount of lithium contributed by the impregnation solution described below.

Preferably, the catalyst comprises 10 to 1000 ppm by weight of sodium, relative to the total weight of the catalyst. It is preferred that the catalyst comprises 20 to 500 ppm by weight of sodium, most preferably 30 to 250 ppm by weight of sodium. The amount of sodium is determined by the amount of sodium contributed by the porous support and the amount of sodium contributed by the impregnation solution described below.

The catalyst may also include a Group IIA alkaline earth metal or a mixture of two or more Group IIA alkaline earth metals. Suitable alkaline earth metal promoters include, for example, beryllium, magnesium, calcium, strontium, and barium or combinations thereof. The amounts of alkaline earth metal promoters can be used in amounts similar to those used for the alkali or transition metal promoters.

The catalyst may also include a promoting amount of a main group element or a mixture of two or more main group elements. Suitable main group elements include any of the elements in Groups IIIA (boron group) to VIIA (halogen group) of the Periodic Table of the Elements. For example, the catalyst can include a promoting amount of sulfur, phosphorus, boron, halogen (e.g., fluorine), gallium, or a combination thereof.

The catalyst may also include a promoting amount of a rare earth metal or a mixture of two or more rare earth metals. The rare earth metals include any of the elements having an atomic number of 57-103. Some examples of these elements include lanthanum (La), cerium (Ce), and samarium (Sm). The amount of rare earth metal promoters can be used in amounts similar to those used for the transition metal promoters.

Further provided is a process for producing an alkylene oxide by gas-phase oxidation of an alkylene, comprising reacting an alkylene and oxygen in the presence of a catalyst as defined in any one of the preceding claims. It is understood that all embodiments of the catalyst also apply to the process for producing ethylene oxide in the presence of the catalyst, where applicable.

Suitable alkenes include ethylene, propylene, 1-butene, isobutene, 2-butene, 1,3-butadiene and 1,9 decadiene, or combinations thereof. Preferably, the process for producing an alkylene oxide by gas-phase oxidation of an alkylene is a process for producing ethylene oxide by gas-phase oxidation of ethylene.

The epoxidation can be carried out by all processes known to those skilled in the art. It is possible to use all reactors which can be used in the ethylene oxide production processes of the prior art; for example externally cooled shell-and-tube reactors (cf. Ullmann's Encyclopedia of Industrial Chemistry, 5th edition, vol. A-10, pp. 117-135, 123-125, VCH-Verlagsgesellschaft, Weinheim 1987) or reactors having a loose catalyst bed and cooling tubes, for example the reactors described in DE-A 3414717, EP 0082609 and EP-A 0339748.

The oxidation is preferably carried out in at least one tube reactor, preferably in a shell-and-tube reactor. On a commercial scale, ethylene epoxidation is preferably carried out in a multi-tube reactor that contains several thousand tubes. The catalyst is filled into the tubes, which are placed in a shell that is filled with a coolant. In commercial applications, the internal tube diameter is typically in the range of 20 to 40 mm (see, e.g., US 4,921,681) or more than 40 mm (see, e.g., WO2006/102189).

To produce ethylene oxide from ethylene and oxygen, it is possible to carry out the reaction under conventional reaction conditions as described, for example, in DE-A 2521906, EP-A 0 014 457, DE-A 2300512, EP-A 0 172 565, DE-A 2454972, EP-A 0 357 293, EP-A 0 266 015, EP-A 0 085 237, EP-A 0 082 609 and EP-A 0 339 748. Inert gases such as nitrogen or gases which are inert under the reaction conditions, e.g. steam, methane, and also optionally reaction moderators, for example halogenated hydrocarbons such as ethyl chloride, vinyl chloride or 1,2-dichloroethane can additionally be mixed into the reaction gas comprising ethylene and molecular oxygen.

The oxygen content of the reaction gas is advantageously in a range in which no explosive gas mixtures are present. A suitable composition of the reaction gas for producing ethylene oxide can, for example, comprise an amount of ethylene in the range from 10 to 80% by volume, preferably from 20 to 60% by volume, more preferably from 25 to 50% by volume and particularly preferably in the range from 25 to 40% by volume, based on the total volume of the reaction gas. The oxygen content of the reaction gas is advantageously in the range of not more than 10% by volume, preferably not more than 9% by volume, more preferably not more than 8% by volume and very particularly preferably not more than 7.5% by volume, based on the total volume of the reaction gas.

The reaction gas preferably comprises a chlorine-comprising reaction moderator such as ethyl chloride, vinyl chloride or 1,2-dichloroethane in an amount of from 0 to 15 ppm by weight, preferably in an amount of from 0.1 to 8 ppm by weight, based on the total weight of the reaction gas. The remainder of the reaction gas generally comprises hydrocarbons such as methane and also inert gases such as nitrogen. In addition, other materials such as steam, carbon dioxide or noble gases can also be comprised in the reaction gas.

The concentration of carbon dioxide in the feed (i.e. the gas mixture fed to the reactor) typically depends on the catalyst selectivity and the efficiency of the carbon dioxide removal equipment. Carbon dioxide concentration in the feed is preferably at most 3 vol.-%, more preferably less than 2 vol.-%, most preferably less than 1 vol.-%, relative to the total volume of the feed. An example of carbon dioxide removal equipment is provided in US 6,452,027.

The above-described constituents of the reaction mixture may optionally each have small amounts of impurities. Ethylene can, for example, be used in any degree of purity suitable for the gas-phase oxidation according to the invention. Suitable degrees of purity include, but are not limited to, "polymer-grade" ethylene, which typically has a purity of at least 99%, and "chemical-grade" ethylene which typically has a purity of less than 95%. The impurities typically comprise, in particular, ethane, propane and/or propene.

The reaction or oxidation of ethylene to ethylene oxide is usually carried out at elevated catalyst temperatures. Preference is given to catalyst temperatures in the range of 150 to 350 °C, more preferably 180 to 300 °C, particularly preferably 190 to 280 °C and especially preferably 200 to 280 °C. The present invention therefore also provides a process as described above in which the oxidation is carried out at a catalyst temperature in the range 180 to 300 °C, preferably 200 to 280 °C. Catalyst temperature can be determined by thermocouples located inside the catalyst bed. As used herein, the catalyst temperature or the temperature of the catalyst bed is deemed to be the weight average temperature of the catalyst bodies.

The reaction according to the invention (oxidation) is preferably carried out at pressures in the range of 5 to 30 bar. All pressures herein are absolute pressures, unless noted otherwise. The oxidation is more preferably carried out at a pressure in the range of 5 to 25 bar, such as 10 bar to 20 bar and in particular 14 bar to 20 bar. The present invention therefore also provides a process as described above in which the oxidation is carried out at a pressure in the range of 14 bar to 20 bar.

The process according to the invention is preferably carried out under conditions conducive to obtain a reaction mixture containing at least 2.0 vol.-% of ethylene oxide. In other words, the ethylene oxide outlet concentration (ethylene oxide concentration at the reactor outlet) is preferably at least 2.0 vol.-%. The ethylene oxide outlet concentration is more preferably in the range of 2.2 to 4.0 vol.-%, most preferably in the range of 2.9 to 3.5 vol.-%.

The oxidation is preferably carried out in a continuous process. If the reaction is carried out continuously, the GHSV (gas hourly space velocity) is, depending on the type of reactor chosen, for example on the size/cross-sectional area of the reactor, the shape and size of the catalyst, preferably in the range from 800 to 10,000/h, preferably in the range from 2,000 to 8,000/h, more preferably in the range from 2,500 to 6,000/h, most preferably in the range from 4,500 to 5,500/h, where the values indicated are based on the volume of the catalyst.

In one embodiment, the EO-space-time-yield measured is greater than 180 kg_{EO}/(m³_{cat}h), preferably to an EO-space-time-yield of greater than 200 kg_{EO}/(m³_{cat}h), such as greater than 250 kg_{EO}/(m³_{cat}h), greater than 280 kg_{EO}/(m³_{cat}h), or greater than 300 kg_{EO}/(m³_{cat}h). Preferably the EO-space-time-yield measured is less than 500 kg_{EO}/(m³_{cat}h), more preferably the EO-space-time-yield is less than 350 kg_{EO}/(m³_{cat}h).

The production of ethylene oxide from ethylene and oxygen can advantageously be carried out in a recycle process. After each pass, the newly formed ethylene oxide and the by-products formed in the reaction are removed from the product gas stream. The remaining gas stream is supplemented with the required amounts of ethylene, oxygen and reaction moderators and reintroduced into the reactor. The separation of the ethylene oxide from the product gas stream and its work-up can be carried out by customary methods of the prior art (cf. Ullmann's Encyclopedia of Industrial Chemistry, 5th edition, vol. A-10, pp. 117-135, 123-125, VCH-Verlagsgesellschaft, Weinheim 1987).

The invention will be described in more detail by the subsequent examples.

### Examples

### A. Methods

### Method 1: Mercury Porosimetry

Mercury porosimetry was performed using a Micrometrics AutoPore IV 9500 mercury porosimeter (140 degrees contact angle, 485 dynes/cm Hg surface tension, 60000 psia max head pressure). The Hg porosity is determined in accordance with DIN 66133.

### Method 2: BET Surface Area

The BET surface area was determined in accordance with DIN ISO 9277.

### Method 3: Mercury Porosimetry

Mercury porosimetry was performed using a Micrometrics AutoPore IV 9500 mercury porosimeter (140 degrees contact angle, 485 dynes/cm Hg surface tension, 60000 psia max head pressure). The Hg porosity is determined in accordance with DIN 66133.

### Method 4: Analysis of Silver Content

Shaped catalyst bodies were crushed and pulverized so as to obtain homogenized samples. 300 to 700 mg of pulverized catalyst bodies were weighed into a titrator (888 Titrando, Metrohm). The sample was brought into contact with 10 mL of a mixture of 65% HNO₃ : H₂O (1:1) at boiling temperature. The obtained mixture was diluted with 150 mL of H₂O and titrated with a 0.1 M solution of ammonium thiocyanate, using a silver electrode.

### Method 5: Side Crush Strength

The side crush strength was determined using an apparatus of the "Z 2.5 / T 919" type supplied by Zwick Roll (Ulm), stamp size: 12.7 mm × 12.7 mm. Based on measurements of 25 randomly selected shaped bodies, average values were calculated. The measurements were performed along two directions - along the side and along the diagonal. In the measurement along the diagonal, the force is exerted along an axis running through a first outer passageway, the central passageway and a second outer passageway opposite to the first outer passageway. In the measurement along the side, the force is exerted along two axes each running through a two outer passageways.

### Method 6: Attrition Loss

Attrition loss was determined according to ASTM D4058-96.

### Porous Supports

Porous supports A, B were alumina supports and comprised Si, Ca, Mg, Na, K and Fe as chemical impurities. The supports were obtained from EXACER s.r.l. (Via Puglia 2/4, 41049 Sassuolo (MO), Italy), under the lot number COM 32/19 (support A) and COM 55/19 (support B). The properties of supports A and B are listed in Table 1.

Each support had a tetralobe shape with five passageways extending between its two face side surfaces in a quincunx arrangement. Both supports had a bimodal pore size distribution with the first log differential pore volume distribution peak in the range of 0.4 to 0.6 µm and the second log differential pore volume distribution peak in the range of 10 to 30 µm, as measured by Hg intrusion according to DIN 66133.

**Table 1: Support Properties**

| | Support A | Support B |
|---|---|---|
| Amounts of Impurities in Alumina Supports | | |
| Si [ppm] | 600 | 420 |
| Ca [ppm] | 400 | 250 |
| Mg [ppm] | 200 | 115 |
| Na [ppm] | 125 | 80 |
| K [ppm] | 180 | 200 |
| Fe [ppm] | 100 | 140 |

| Physical Properties | | |
|---|---|---|
| BET surface area [m²/g] | 1.95 | 2.07 |
| Total pore volume by Hg intrusion [mL/g] | 0.55 | 0.52 |
| Side crush strength [N] along diagonal | n.d. * | 95 |
| Side crush strength [N] along side | 96 | 116 |
| Attrition loss [wt%] | n.d. * | 16 |

| | | |
|---|---|---|
| * n.d. = not determined | | |

### Example 1 - Preparation of Catalysts

Shaped catalyst bodies according to Table 2 below were produced by impregnating refractory support A with a silver impregnation solution.

**Table 2: Catalyst Composition (Ag-contents are reported in percent by weight of total catalyst, promoter values are reported in parts per million by weight of total catalyst)**

| Example | Support | Ag_{CAT} [wt-%] | Li_{CAT} [ppm] | S_{CAT} [ppm] | W_{CAT} [ppm] | CS_{CAT} [ppm] | RE_{CAT} [ppm] | K_{ADD ***} [ppm] | K_{CAT ***} [ppm] |
|---|---|---|---|---|---|---|---|---|---|
| 1-1 | A | 20.5 * / 20.0 ** | - | - | - | - | - | 63 | 206 |
| 1-2 | A | 20.5 * / 19.3 ** | - | - | - | - | - | 63 | 206 |
| 1-3 | B | 21.1 */ 20.2 ** | - | - | - | - | - | 65 | 223 |
| 1-4 | B | 21.1 */ 18.9 ** | - | - | - | - | - | 65 | 223 |

| | | | | | | | | | |
|---|---|---|---|---|---|---|---|---|---|
| * calculated Ag values, ** analyzed Ag values; all promoter values are calculated values; *** K_{ADD} is understood to mean the amount of potassium added during impregnation and does not include the amount of potassium comprised in the alumina support prior to impregnation; **** K_{CAT} is understood to mean the total amount of potassium in the catalyst | | | | | | | | | |

### 2.1 Production of a Silver Impregnation Solution

A silver impregnation solution was prepared according to Production Example 1 of WO 2019/154863 A1. The silver impregnation solution had a density of 1.529 g/mL, a silver content of 29.3 wt-% and a potassium content of 90 ppm.

### 2.2. Preparation of Catalyst 1-1

140.3 g of support A were placed into a 2 L glass flask, and the flask with the support was pre-heated at 60 °C for 2 h. Subsequently, the flask was attached to a rotary evaporator and a vacuum of 80 mbar was applied while the temperature was maintained at 55 °C using a water bath. The rotary evaporator system was set in rotation of 10 rpm. 123.5 g of silver impregnation solution prepared according to Example 2.1 was added to support A over a period of 60 min, while maintaining a vacuum of 80 mbar at 55 °C.

After addition of the silver impregnation solution, the rotary evaporator system was cooled down to room temperature (23 °C) and was continued to rotate under a vacuum of 80 mbar for another 15 min. The impregnated support was subsequently left in the apparatus at room temperature (23 °C) and atmospheric pressure for 1 h and mixed gently every 15 min.

The weight loss of the contents of the flask during impregnation was determined to be 5.27 g, which corresponds to a weight loss of 4.3 wt.-%, relative to the weight of aqueous impregnation solution.

The impregnated support was divided into two aliquots of about 133 g each and placed on a net, forming a monolayer. The two aliquots of impregnated material were each heated under a nitrogen flow (23 Nm³/h) to a temperature of 290 °C at a heating rate of about 30 K/min. The temperature was subsequently maintained at 290 °C for 8 min to yield a total amount of 175.4 g of catalyst 1-1. The measured silver content in catalyst 1-1 was 20.0 wt.-%.

### 2.3. Preparation of Catalyst 1-2 (Reference Example)

140.2 g of support A were placed into a 2 L glass flask. The flask was attached to a rotary evaporator and a vacuum of 80 mbar was applied. The rotary evaporator system was set in rotation of 10 rpm. 123.4 of silver impregnation solution prepared according to Example 2.1 were added to support A over a period of 60 min while maintaining a vacuum of 80 mbar under vacuum of 80 mbar at room temperature (23 °C).

After addition of the silver impregnation solution, the rotary evaporator system was continued to rotate under a vacuum of 80 mbar for another 15 min. The impregnated support was then left in the apparatus at room temperature (23 °C) and atmospheric pressure for 1 h and mixed gently every 15 min. During the impregnation, excess silver impregnation solution was present in the glass flask, i.e. silver impregnation solution not absorbed by the support bodies.

The weight loss of the contents of the flask during impregnation was determined to be 1.5 g, which corresponds to a weight loss of 1.2 wt.-%, relative to the weight of aqueous impregnation solution.

The impregnated support was divided into two aliquots of about 133 g each and placed on a net, forming a monolayer. The two aliquots of impregnated material were each heated under a nitrogen flow (23 Nm³/h) to a temperature of 290 °C at a heating rate of about 30 K/min. The temperature was subsequently maintained at 290 °C for 8 min to yield a total amount of 173.3 g of reference catalyst 1-2. The measured silver content in catalyst 1-2 was 19.3 wt%.

From the comparison of examples 2.2 and 2.3, it is evident that the inventive process yields a higher silver content in the obtained catalyst than a comparative process.

### 2.4. Preparation of Catalyst 1-3

140.8 g of support B were placed into a 2 L glass flask, and the flask with the support was pre-heated at 60 °C for 2 h. Subsequently, the flask was attached to a rotary evaporator and a vacuum of 80 mbar was applied while the temperature was maintained at 55 °C using a water bath. The rotary evaporator system was set in rotation of 10 rpm. 128.5 g of silver impregnation solution prepared according to Example 2.1 was added to support B over a period of 60 min, while maintaining a vacuum of 80 mbar at 55 °C.

After addition of the silver impregnation solution, the rotary evaporator system was continued to rotate under a vacuum of 80 mbar for another 15 min at 55 °C. The impregnated support was subsequently left in the apparatus at room temperature (23 °C) and atmospheric pressure for 1 h and mixed gently every 15 min.

The weight loss of the contents of the flask during impregnation was determined to be 9.59 g, which corresponds to a weight loss of 7.5 wt.-%, relative to the weight of aqueous impregnation solution.

The impregnated support was divided into two aliquots of about 133 g each and placed on a net, forming a monolayer. The two aliquots of impregnated material were each heated under a nitrogen flow (23 Nm³/h) to a temperature of 290 °C at a heating rate of about 30 K/min. The temperature was subsequently maintained at 290 °C for 8 min to yield a total amount of 176.7 g of catalyst 1-3. The measured silver content in catalyst 1-3 was 20.2 wt.-%.

### 2.5. Preparation of Catalyst 1-4 (Reference Example)

140.7 g of support B were placed into a 2 L glass flask. The flask was attached to a rotary evaporator and a vacuum of 80 mbar was applied. The rotary evaporator system was set in rotation of 10 rpm. 128.4 of silver impregnation solution prepared according to Example 2.1 were added to support B over a period of 60 min while maintaining a vacuum of 80 mbar under vacuum of 80 mbar at room temperature (23 °C).

After addition of the silver impregnation solution, the rotary evaporator system was continued to rotate under a vacuum of 80 mbar for another 15 min. The impregnated support was then left in the apparatus at room temperature (23 °C) and atmospheric pressure for 1 h and mixed gently every 15 min. During the impregnation, excess silver impregnation solution was present in the glass flask, i.e. silver impregnation solution not absorbed by the support bodies.

The weight loss of the contents of the flask during impregnation was determined to be 1.76 g, which corresponds to a weight loss of 1.4 wt.-%, relative to the weight of aqueous impregnation solution.

The impregnated support was divided into two aliquots of about 133 g each and placed on a net, forming a monolayer. The two aliquots of impregnated material were each heated under a nitrogen flow (23 Nm³/h) to a temperature of 290 °C at a heating rate of about 30 K/min. The temperature was subsequently maintained at 290 °C for 8 min to yield a total amount of 170.8 g of comparative catalyst 1-4. The measured silver content in catalyst 1-4 was 18.9 wt%.

From the comparison of examples 2.4 and 2.5, it is evident that the inventive process yields a higher silver content in the obtained catalyst than a comparative process.

## Claims

1. A process for producing an epoxidation catalyst, comprising:
i) placing a porous support under reduced pressure, the porous support having an Hg pore volume in the range of 0.4 to 3.0 mL/g, as determined by mercury porosimetry;
ii) gradually adding to the porous support a volume of aqueous silver impregnation solution over a period of at least 15 min at a temperature of 40 to 150 °C, while maintaining reduced pressure; the volume of aqueous silver impregnation solution added per gram of porous support being greater than the Hg pore volume of the porous support;
iii) maintaining the reduced pressure to remove volatiles to a weight loss of at least 3 wt.-%, relative to the weight of aqueous silver impregnation solution;
iv) calcining the impregnated porous support at a temperature of 200 to 800 °C.

2. The process of claim 1, comprising pre-heating the porous support to a temperature of 40 to 200 °C prior to addition of the silver impregnation solution.

3. The process of claim 1 or 2, comprising agitation of the porous support during steps ii) and iii).

4. The process of any one of the preceding claims, wherein the porous support comprises individual shaped bodies.

5. The process of any one of the preceding claims, wherein the porous support comprises at least 50 wt.-% of alpha-alumina, based on the total weight of the porous support.

6. The process of any one of the preceding claims, wherein the porous support has a BET surface area of 0.5 to 10 m²/g.

7. The process of any one of the preceding claims, wherein steps i) to iv) are repeated.

8. The process of any one of the preceding claims, wherein the volume(s) and concentration of aqueous silver impregnation solution are adapted to yield an epoxidation catalyst comprising 15 to 70 wt.-% of silver, preferably 20 to 60 wt.-% of silver, more preferably 20 to 50 wt.-% of silver, such as 25 to 35 wt.-%, relative to the total weight of the catalyst.

9. A process for producing an alkylene oxide by gas-phase oxidation of an alkylene, comprising reacting an alkylene and oxygen in the presence of a catalyst as defined in any one of the preceding claims.

10. The process of claim 9 for producing ethylene oxide by gas-phase oxidation of ethylene, comprising reacting ethylene and oxygen in the presence of a catalyst as defined in any one of the claims 1 to 8.
